# EUROPÄISCHE PATENTANMELDUNG

(11) **EP 0 687 687 A2**
(43) Veröffentlichungstag der Anmeldung: **20.12.1995**
(21) Anmeldenummer: 95107998.7
(22) Anmeldetag: 24.05.1995
(51) Int. Cl.: C07K 14/745, A61K 38/36

(54) **Pharmazeutische Zusammensetzung zur Vorbeugung und Behandlung von Blutgerinnungsstörungen**

(30) Priorität: 27.05.1994 DE 4418635
(71) Anmelder: IMMUNO Aktiengesellschaft, A-1221 Wien (AT)
(72) Erfinder: Eibl, Johann, Dr., A-1180 Wien (AT); Schwarz, Hans Peter, Dr., A-1180 Wien (AT); Varadi, Katalin, Dr., A-1230 Wien (AT)
(74) Vertreter: Kolb, Helga, Dr. Dipl.-Chem.

(57) **Zusammenfassung**

Es wird eine pharmazeutische Zusammensetzung zur Vorbeugung und Behandlung von Blutgerinnungsstörungen beschrieben, die dadurch gekennzeichnet ist, daß sie den Faktor V als natives Protein, Derivat und/oder Fragment davon, und Protein S in einem geeigneten pharmazeutischen Träger enthält.

## Beschreibung

Die Erfindung betrifft eine pharmazeutische Zusammensetzung zur Vorbeugung und Behandlung von Blutgerinnungsstörungen, die den Faktor V, dessen Derivat und/oder Fragment, und Protein S enthalten.

Unter Blutgerinnung versteht man den Vorgang der Umwandlung des flüssigen Blutes in den sogenannten Blutkuchen, der eine gallertartige Masse darstellt, die die Abdichtung verletzter Blutgefäße durch Pfropfbildung ermöglicht. Die Umwandlung des im Plasma vorhandenen löslichen Fibrinogens in den fasereriggallertigen Gerinnungsstoff, das sogenannte Fibrin, erfolgt in einem mehrstufigen Prozess (sogenannte Blutgerinnungs-Kaskade), an dem mindestens 15 verschiedene, mit römischen Ziffern gekennzeichnete Blutgerinnungsfaktoren beteiligt sind, von denen jeder, wenn aktiviert, seinerseits wieder die jeweilst nächste inaktive Vorstufe aktiviert. Unter diesen Aktivatoren befinden sich mehrere Serin-Proteinasen (wie Kallikrein, Thrombin, und die aktivierten Faktoren VII, IX, X, XI und XII). Ein aktiviertes Gerinnungsfaktor wird auch mit "a" (im Index) gekennzeichnet. Durch die relativ große Zahl der Aktivierungsschritte in dieser Blutgerinnungskaskade und die in jeder Stufe vorhandene katalytische Wirkung wird eine Verstärkung dahingehend erreicht, daß selbst bei sehr geringer Menge an initiierendem Faktor in möglichst kurzer Zeit eine ausreichende Menge an Fibrin vorhanden ist. Außerdem sind an der Gerinnungswirkung auch die zwischen den entstehenden Fibrinfasern eingeschlossenen Blutzellen beteiligt.

Man unterscheidet prinzipiell zwischen 2 verschiedenen Wegen der Aktivierung der Blutgerinnung, die aber beide in einen gemeinsamen Weg münden.

Der intrinsische Blutgerinnungsweg betrifft die Blutgerinnungskomponenten, die normalerweise im Blut zirkulieren und kann eingeleitet werden, wenn Blut mit unphysiologischen Oberflächen, z.B. mit Glas oder Keramik, in Berührung kommt, wobei die beiden Proteine Kininogen und Kallikrein beteiligt sind. Nacheinander werden dann die Faktoren XII, XI, IX und X aktiviert, wobei beim letztgenannten Schritt der Aktivierung von X durch IXa der bei der Bluterkrankheit Hämophilie A fehlende Faktor VIII nötig ist.

Beim extrinsischen Blutgerinnungsweg wird mit der Verletzung von Blutgefäßen ein Gewebefaktor (tissue factor, der ein Lipoprotein ist) ausgeschüttet sowie der sogenannte Faktor VII aktiviert, die dann beide zusammen den vorstehend genannten Faktor X aktivieren. Der anschließende gemeinsame Weg, der sowohl bei der intrinsischen Blutgerinnung als auch bei der extrinsischen Blutgerinnung danach beschritten wird, ist dann der folgende:
Faktor Xa und Faktor Va aktivieren Prothrombin (den Faktor II) an der Thrombocyten-Membran zu Thrombin, und dieses setzt durch Abspaltung von Peptiden aus Fibrinogen (Faktor I) Fibrin-Monomere frei, die in der Lage sind, zu Fibrin-Fasern zu polymerisieren. Durch den aktivierten Faktor XIII kommt es schließlich dann zur Quervernetzung und somit zur Stabilisierung der Fibrinfasern. Der intrinsische und der extrinsische Blutgerinnungsweg sind insofern miteinander verflochten, als der aktivierte Faktor VII zusammen mit dem Gewebefaktor auch den Faktor IX aktiviert, während z.B. Kallikrein auch den extrinsischen Weg stimulieren kann. Außer den genannten Faktoren ist bei einigen Schritten die Anwesenheit von Phospholipiden und Calciumionen nötig, die an 4-Carboxy-L-glutaminsäurereste binden. Die Carboxylierung von L-Glutamat in diesen Proteinen ist dabei abhängig von Vitamin K.

Die Inhibition der Blutgerinnung erfolgt durch aktiviertes Protein C (ebenfalls eine Serin-Proteinase), das die Faktoren Va und VIIIa proteolytisch inaktiviert. Antithrombin III (ein Serin-Proteinase-Inhibitor) trägt ebenfalls zur notwendigen Kontrolle der Blutgerinnung bei, indem es Thrombin und die anderen beteiligten Proteinasen irreversibel hemmt. Die Auflösung nicht mehr benötigter Gerinnungspfropfen wird dann durch die Serin-Proteinase Plasmin bewerkstelligt, die Fibrin-Fasern proteolytisch abbauen (vgl. z.B. Römpp Chemielexikon, 9. Auflage (1989) 463; Römmp Lexikon Biotechnologie (1992) 133).

Das Protein C (Autoprothrombin II A, Faktor XIV) ist ein Vitamin K-abhängiges Glykoprotein, das in der Leber synthetisiert wird und im Plasma in einer Konzentration von 4 µg/ml als inaktives Zymogen zirkuliert. Es wird an der Gefäßwandoberfläche (dem Endothel) durch den Thrombin-Thrombomodulin-Komplex in die aktive Serinprotease (aktiviertes Protein C, APC) überführt, und besitzt als aktiviertes Protein C (aktive Serin-Protease) profibrinolytische Eigenschaften. Es wirkt auch antikoagulatorisch, weil es den Faktor Va, den Kofaktor für die Faktor Xa-induzierte Prothrombinaktivierung (Thrombinbildung) und den Faktor VIIIa, den Kofaktor für die Faktor IXa-induzierte Faktor X-Aktivierung, durch Proteolyse abbaut.

Neben Calcium-Ionen und Phospholipiden wirkt ein weiteres Protein, das sogenannte Protein S (Molekulargewicht 84 000) als Kofaktor, das jedoch selbst keine Protease-Aktivität besitzt; Protein S wirkt nicht-enzymatisch als Kofaktor für die antikoagulatorischen und profibrinolytischen Eigenschaften von Protein C. Natives Plasma enthält zwischen 25 mg und 30 mg Protein S pro ml, wobei Protein S in verschiedenen Formen vorkommt. Die Kofaktoraktivität wird der freien Form von Protein S zugeschrieben, während ein Komplex von Protein S mit C4b-bindendem Protein keine Aktivität zeigt.

Es ist bekannt, daß aktiviertes Protein C in dosisabhängiger Weise die Plasmagerinnungszeit verlängert. In einem Protein S-Mangelplasma kann aktiviertes Protein C jedoch seine Funktion nicht entfalten, sondern erst durch einen Zusatz von Protein S wird die Wirkung von aktiviertem Protein C verbessert.

Die Wirkung von Protein S auf aktiviertes Protein C wird beispielsweise beschrieben in Blood Coagulation and Fibrinolysis 3 (1992) 257 bis 261. Es wurde beobachtet, daß eine Erhöhung der Protein S-Konzentration zu einer linearen Verstärkung des antikoagulativen Effektes von aktiviertem Protein C führt. Dabei wurde gefunden, daß die Konzentration von Faktor Va keine Rolle spielte. In einem Faktor V-Mangelplasma wurde beobachtet, daß die antikoagulatorische Wirkung von aktiviertem Protein C verhältnismäßig gering war. Durch einen Zusatz von Protein S konnte jedoch die mangelhafte Wirkung von aktiviertem Protein C verbessert werden.

Der verringerten Wirkung von aktiviertem Protein C werden eine Reihe von Gründen zugeschrieben. In Proc. Natl. Acad. Sci. (PNAS) USA 90 (1993) 1004 bis 1008 wird eine schwache antikoagulatorische Antwort des aktivierten Protein C bei einer Reihe von Patienten beschrieben. Diese Patienten besaßen aber nachweislich keinen Mangel an Protein C, Protein S, Antithrombin III, Plasminogen und anderen relevanten Blutbestandteilen. Es wurde deshalb angenommen, daß ein bislang unbekannter Kofaktor für aktiviertes Protein C für dieses Krankheitsbild verantwortlich sein dürfte. Ein Mangel an diesem Kofaktor für aktiviertes Protein C kann daher zu thromboembolischen Krankheitszuständen führen.

Es wurde gefunden, daß eine entsprechende Kofaktor-Aktivität mit dem Gerinnungsfaktor V assoziert ist. In Pro. Natl. Acad. Sci. (PNAS) USA 91 (1994) 1396 bis 1400 wird dem Faktor V eine antikoagulatorische Kofaktor-Aktivität für aktiviertes Protein C zugeschrieben.

Der Faktor V ist ein β-Globulin mit einem Molekulargewicht von 330 kD, das über eine limitierte Proteolyse von Thrombin aktiviert und durch aktiviertes Protein C inaktiviert wird. Dabei entstehen spezifische Fragmente. Faktor V ist in seiner aktivierten Form Bestandteil des Prothrombinasekomplexes (bestehend aus Faktor Xa, Faktor Va, Phospholipid, Calciumionen zur Aktivierung von Prothrombin), der für die Thrombinbildung und damit für die Blutgerinnung verantwortlich ist. Damit gilt der Faktor V an sich als ein prokoagulatorischer Gerinnungsfaktor. Der angeborene und vererbare Mangel an Faktor V wird mit einer der Hämophilie ähnlichen Blutungsneigung assoziiert.

Die WO-A-93/10261 beschreibt Verfahren zur Bestimmung von Blutgerinnungsstörungen, die auf einer schwachen Wirkung von aktiviertem Protein C beruhen.

Ein weiteres Verfahren zur Bestimmung der Empfindlichkeit einer Blut- bzw. Plasmaprobe für aktiviertes Protein C ("APC-sensitivity", "APC-response") wird in der US-Anmeldung Serial No. 08/160877 beschrieben. Danach wird in einfacher Weise die Faktor VIII-Aktivität einer Probe, der aktiviertes Protein C zugesetzt wurde, mittels eines chromogenen Assays über Faktor Xa bestimmt und zu der Faktor VIII-Aktivität in Relation gesetzt, die ohne Zusatz von aktiviertem Protein C gemessen wird. Je größer das erhaltene Verhältnis ist, um so höher ist die Empfindlichkeit gegenüber aktiviertem Protein C.

Aufgabenstellung der vorliegenden Erfindung ist es, ein pharmazeutisches Präparat bereitzustellen, mit dem die Wirkung von aktiviertem Protein C normalisiert oder verstärkt werden kann.

Diese Aufgabe wird mit dem Gegenstand der vorliegenden Erfindung gelöst.

Gegenstand der Erfindung ist eine pharmazeutische Zusammensetzung zur Vorbeugung und Behandlung von Blutgerinnungsstörungen, die dadurch gekennzeichnet ist, daß sie den Faktor V als natives Protein, Derivat und/oder Fragment davon, und Protein S in einem geeigneten pharmazeutischen Träger enthält (APC-Enhancer).

Bevorzugte Ausführungsformen dieser Zusammensetzung sind Gegenstand der Ansprüche 2 und 3.

Weiterer Gegenstand ist die Verwendung von Faktor V als natives Protein, dessen Derivat und/oder Fragmente in Kombination mit Protein S und/oder einer Protein S haltigen pharmazeutischen Zusammensetzung zur Vorbeugung und/oder Behandlung von Blutgerinnungsstörungen.

Bevorzugte Ausgestaltungen davon sind Gegenstand der Ansprüche 5 bis 7.

Weiterer Gegenstand ist die Verwendung von Faktor V als natives Protein, dessen Derivat und/oder Fragmente in Kombination mit Protein S zur Herstellung einer pharmazeutischen Zusammensetzung zur Vorbeugung und/oder Behandlung von Blutgerinnungsstörungen.

Bevorzugte Ausgestaltungen davon sind Gegenstand der Ansprüche 9 und 10.

Die Anwendung des Faktors V, dessen Derivat und/oder Fragment und des Protein S oder Protein S haltigen Präparates zur Vorbeugung und Behandlung von Blutgerinnungsstörungen kann dabei gemeinsam, gleichzeitig (aber voneinander getrennt) oder aber auch hintereinander, und zwar in beliebiger Reihenfolge, erfolgen.

Weiterer Gegenstand der Erfindung ist auch ein Set (Kit) zur Herstellung einer erfindungsgemäßen pharmazeutischen Zusammensetzung, die in 2 getrennten Behältern den Faktor V als natives Protein, Derivat und/oder Fragment und in einem oder mehreren weiteren Behältern Protein S und/oder ein Protein S haltiges Präparat enthält, wobei die Behälter gegebenenfalls noch weitere pharmazeutisch annehmbare Träger und Verdünner in Kombination mit den Wirkstoffen, und/oder aber auch getrennt davon in einem separaten Behälter, enthalten können.

In einer bevorzugten Ausführungsform ist die erfindungsgemäße Zusammensetzung im wesentlichen frei von aktiviertem Faktor V. In einer weiteren zweckmäßigen Ausführungsform enthält die erfindungsgemäße Zusammensetzung neben dem Faktor V als natives Protein, Derivat und/oder Fragment zusätzlich Protein C und/oder aktiviertes Protein C. Entsprechend kann bei einer direkten Applikation oder Verwendung der Einzelkomponenten zusätzlich noch Protein C und/oder aktivierter Protein C gemeinsam, gleichzeitig, vor oder nach der Applikation oder Verwendung appliziert oder z.B. zur Herstellung einer pharmazeutischen Zusammensetzung verwendet werden.

Das bevorzugte Verhältnis von Faktor V, als natives Protein, Derivat oder Fragment (Faktor V wirkende Substanz) zu Protein S beträgt erfindungsgemäß 1:2 bis 1:100, am meisten bevorzugt 1:5 bis 1:50 (molares Verhältnis).

Zweckmäßigerweise enthält die erfindungsgemäße Zusammensetzung den Faktor V als natives Protein, Derivat oder Fragment davon in einer Art und Menge, die keine prokoagulatorische Aktivität aufweist.

Unter Blutgerinnungsstörungen werden erfindungsgemäß insbesondere thromboembolische Krankheitszustände verstanden.

Es hat sich nun herausgestellt, daß mit der erfindungsgemäßen pharmazeutischen Zusammensetzung, die eine Kombination des Faktors V und von Protein S enthält, Blutgerinnungsstörungen, insbesondere thromboembolischen Krankheitszuständen, wirksam vorgebeugt werden kann, oder diese wirkungsvoll behandelt werden können.

Ew wurde gefunden, daß eine synergistische Wirkung von Protein S und Faktor V auf die "APC-sensitivity" einer Probe (gemessen nach US Serial No. 08/160877 vorliegt.

Überraschenderweise hat sich gezeigt, daß die antikoagulatorische Wirkung von aktiviertem Protein C durch die erfindungsgemäße Zusammensetzung, also durch Gegenwart des Kofaktors Protein S und des Gerinnungsfaktors V in Form von nativem Protein, Derivat und/oder Fragment davon wesentlich gesteigert werden kann.

Diese Ergebnisse müssen in mehrfacher Hinsicht als überraschend gelten: einerseits konnte die Wirkung von aktiviertem Protein C in einem Faktor V-Mangelplasma allein durch den Zusatz von Protein S normalisiert werden (vgl. Blood Coagulation and Fibrinolysis, l.c.); auf der anderen Seite konnte im Hinblick auf eine APC-Kofaktor-Aktivität von Faktor V höchstens eine additive Wirkung von Protein S und Faktor V erwartet werden. Da der Faktor V auch prokoagulatorische Eigenschaften besitzt, mußte möglicherweise nicht nur ein agonistischer, sondern auch ein antagonistischer Effekt auf die Blutgerinnung erwartet werden.

Zweckmäßigerweise ist die erfindungsgemäße Zusammensetzung im wesentlichen frei von aktiviertem Faktor V. Dadurch läßt sich eine unerwünschte prokoagulatorische Aktivität verhindern. Darüberhinaus wurde festgestellt, daß eine verstärkte Wirkung von aktiviertem Protein C durch Faktor V nur im Falle des

Proteins in seiner inaktiven Form, dessen Derivat und/oder Fragmenten gefunden wird. Der aktivierte Faktor V weist hingegen einen gegenteiligen Effekt auf. Aktiviertes Protein C wird überraschenderweise durch den Faktor Va inhibiert, gezeigt an der Inaktivierung von Faktor VIIIa durch aktiviertes Protein C in Gegenwart von Faktor Va. Bei der Herstellung der erfindungsgemäßen pharmazeutischen Zusammensetzung sollte deshalb besonders darauf geachtet werden, daß ein nicht aktivierter Faktor V oder ein entsprechendes Derivat und/oder Fragment davon in einer Art und Menge gewählt wird, die keine prokoagulatorische Wirkung aufweist. Als Fragment eignet sich insbesondere ein Fragment des Faktors V, welches bei der Aktivierung des Proteins abgespalten wird (sogenanntes Aktivierungspeptid).

Der Anteil an aktiviertem Faktor V an der Gesamtmenge an Faktor V beträgt in der erfindungsgemäßen Zusammensetzung vorzugsweise weniger als 50 Gewichtsprozent, und insbesondere weniger als 30 Gewichtsprozent. In einer besonders bevorzugten Ausführungsform wird die Zusammensetzung deshalb so
hergestellt, z.B. in Gegenwart eines Protease-Inhibitors, daß die Anwesenheit von aktiviertem Faktor V weitgehend ausgeschlossen werden kann.

Zusätzlich kann die erfindungsgemäße Zusammensetzung Protein C und/oder aktiviertes Protein C enthalten; dadurch ist eine gleichzeitige Behandlung eines angeborenen oder erworbenen Protein C-Mangelzustandes möglich.

Die erfindungsgemäße Zusammensetzung kann unter Verwendung von aus Blutplasma gewonnenen Proteinen oder von rekombinanten Proteinen und /oder Polypeptiden (auch Protein S-Polypeptide mit einer APC-Kofaktor-Aktivität) hergestellt werden.

Derartige Proteine sind entweder im Handel erhältlich oder können nach an sich üblichen Methoden hergestellt werden (vgl. z.B. EP-A-0406216, EP-A-0255771, EP-0244834).

Unabhängig davon, ob die Proteine aus Blutplasma gewonnen oder unter Verwendung eines Zellkulturverfahrens hergestellt werden, ist es zweckmäßig, Ausgangsmaterialien bzw. Proteine zu verwenden, die zur Inaktivierung von infektiösen Agentien, wie z.B. Viren, Viroiden und Virionen, auf die dafür übliche Weise behandelt wurden.

Die erfindungsgemäße pharmazeutische Zusammensetzung wird, wenn erforderlich, unter Verwendung geeigneter und für derartige Zubereitungen üblicher Hilfs-, Träger und /oder Zusatzstoffe hergestellt, die insbesondere für die Verabreichung an Menschen unbedenklich sind. Die verwendeten Hilfs-Träger und/oder Zusatzstoffe richten sich insbesondere nach dem beabsichtigten Verabreichungsweg. Die pharmazeutischen Zusammensetzungen können in dafür üblichen Formen, z.B. als Tabletten, Dragees, Kapseln, Suppositorien, Syrupen, Injektionslösungen, Infusionslösungen usw. vorliegen; vorzugsweise liegen sie in einer zur intravenösen Verabreichung geeigneten Form vor.

In einer Ausführungsform kann die erfindungsgemäße Zusammensetzung auch als Set (Kit) bereitgestellt werden, in dem die Komponenten voneinander getrennt aufbewahrt werden, und erst zum Zeitpunkt der Anwendung kombiniert werden. Ein erfindungsgemäßes Set zur Herstellung der pharmazeutischen Zusammensetzung enthält z.B. in einem Behälter (a) den Faktor V, dessen Derivat und/oder Fragment, und in einem zweiten Behälter (b) Protein S, und die gegebenenfalls zur Herstellung der pharmazeutischen Zusammensetzung erforderlichen Hilfs-, Träger und/oder Zusatzstoffe können zusammen oder in einzelnen Komponenten aufgeteilt in einem der Behälter (a) und/oder (b) enthalten sein, oder aber auch noch in einem zusätzlichen weiteren Behälter (c).

Die Vermischung der Inhaltsstoffe der Behälter (a), (b) und gegebenenfalls (c) kann auf eine an sich übliche Weise erfolgen, und erfolgt zweckmäßigerweise kurz vor der Applikation.

In einer Ausführungsform der Erfindung ist es auch möglich, die einzelnen Wirkstoffe der erfindungsgemäßen pharmazeutischen Zusammensetzung einzeln zu applizieren, und zwar entweder gleichzeitig oder, in beliebiger Reihenfolge, hintereinander. So kann z.B. Faktor V, dessen Derivat und/oder Fragment gleichzeitig zusammen mit dem Bestandteil Protein S appliziert werden, oder aber zunächst Faktor V, dessen Derivat und/oder Fragment, und danach das Protein S, oder aber auch in umgekehrter Reihenfolge. Die Verabreichung jedes der Bestandteile erfolgt dabei zweckmäßig ebenfalls, insbesondere abhängig von der Applikation, in Gegenwart eines dafür geeigneten Hilfs-, Träger und/oder Zusatzstoffes. Die Erfindung wird nun in den nachfolgenden Beispielen näher erläutert, ohne sie darauf zu beschränken.

### Beispiel 1

### Verfahren zur Bestimmung auf APC-Sensitivity (APC-Response) (gemäß US Serial No. 08/160877)

### I. Basisverfahren zur Messung der APC-Response im Plasma

50 µl Plasma oder Probe - 1/20 verdünnt in FVIII
Verdünnungspuffer (ImmunochromR FVIII:C, Immuno AG)
50 µl Puffer oder APC (2 U/ml)
100 µl Reagens A (Phospholipid, ImmunochromR FVIII:C, Immuno
AG)
100 µl Reagens B (Aktivatoren + FX, ImmunochromR FVIII:C,
Immuno AG)
------------------------------------------------------------
5 min/37 °C Inkubation
------------------------------------------------------------
-200 µl Chromogensubstrat (enthaltend EDTA, um weitere Xa
Aktivierung zu stoppen) 4 mmol/l CH3OCO-D-CHA-GLY-ARG-pNA-
AcOH
(ImmunochromR FVIII:C, Immuno AG) verdünnt 1:3 in Faktor
VIII-Reaktionspuffer (Immunochrom FVIII:C, Immuno AG)
------------------------------------------------------------
-
5 min/37 °C Inkubation
------------------------------------------------------------
-
mit 100 µl Essigsäure (50 %) wird die Reaktion gestoppt
------------------------------------------------------------
-Die Extinktion wird bei 405 nm gemessen (A405)

Auswertung: Verhältnis-A 405 (Puffer) zu A405 (APC) = APC-
Response

Die Wirkung verschiedener Zusätze auf die APC-Response wurde ebenfalls nach diesem Verfahren wie folgt untersucht.

Die zu untersuchenden Plasmaproben wurden mit Puffer (9+1) gemischt, oder Protein S, oder FV oder FVa, oder mit irgendeinem anderen Reagens, das einen Einfluß auf die durch APC beeinflußte FVIII Inaktivierung haben könnte. Die die Zusätze enthaltende Plasmaprobe wird insgesamt 1/20 verdünnt, und jeweils ohne und mit APC-Zusatz getestet, um das Verhältnis (APC-Response) zu bestimmen.

### Ia. Verfahren zur Messung der Wirkung von APC auf gereinigten Faktor VIII (FVIII)

Es wurde nach der gleichen obigen Methode I verfahren, aber anstelle der Plasmaprobe gereinigter FVIII gemessen. Die Endkonzentration FVIII beträgt ungefähr 0.05 U/ml. Wenn die Wirkung einer oder mehrerer anderer Faktoren getestet wurde, wurden 1 U/ml FVIII im Verhältnis 9+1 oder 8+1+1 mit den Additiven oder Puffern vorgemischt, zu insgesamt 1/20 verdünnt und mit und ohne APC-Zusatz getestet. Nach diesem System ist es auch möglich, die FVIII-Konzentration zu variieren, und andere als die 1/20-Verdünnung zu verwenden, und die APC-Konzentration zu variieren.

Die nach den Methoden des vorstehenden Beispiels 1 erhaltenen Ergebnisse sind nachfolgend tabellarisch und in Form von Grafiken aufgezeigt.

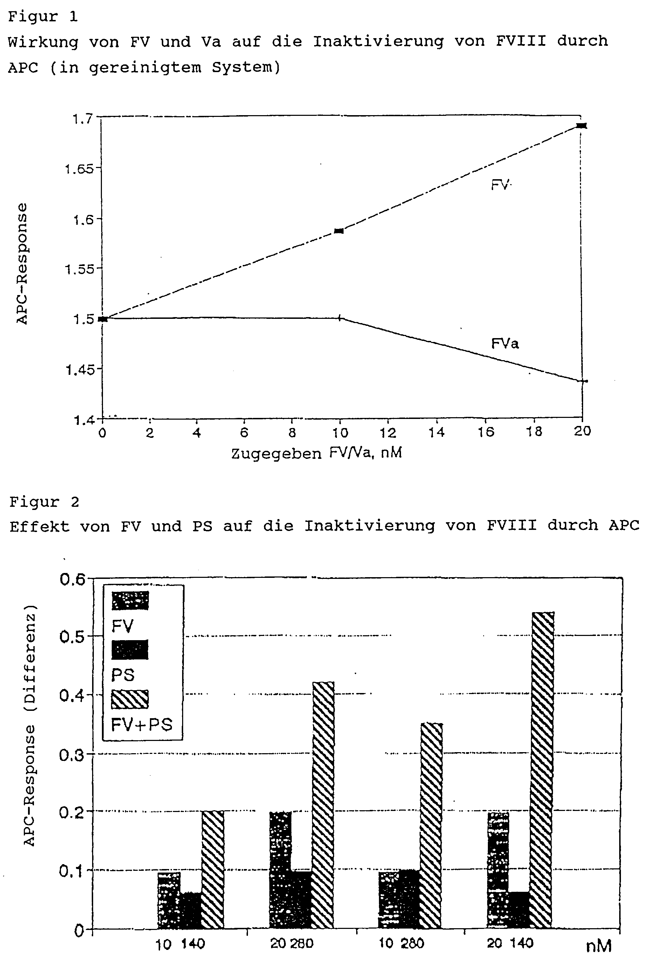

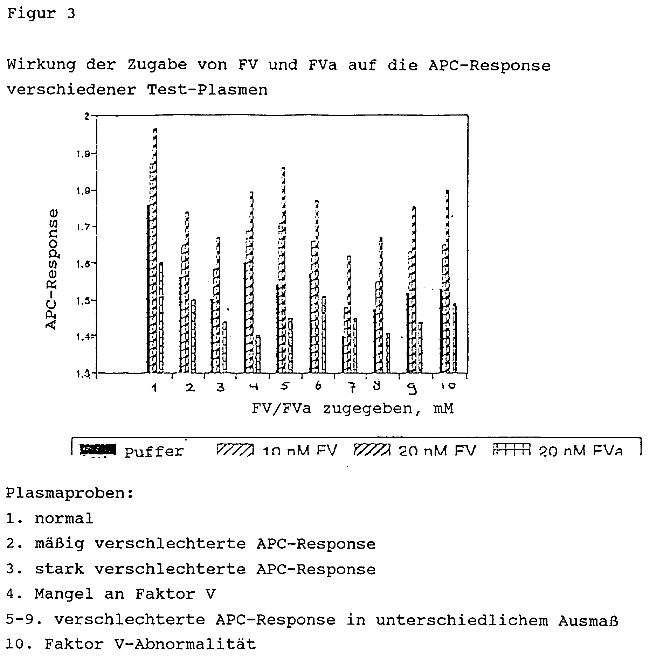

## Patentansprüche

1. Pharmazeutische Zusammensetzung zur Vorbeugung und Behandlung von Blutgerinnungsstörungen, dadurch gekennzeichnet, daß sie den Faktor V als natives Protein, Derivat und/oder Fragment davon, und Protein S in einem geeigneten pharmazeutischen Träger enthält.

2. Zusammensetzung nach Anspruch 1, dadurch gekennzeichnet, daß sie im wesentlichen frei ist von aktiviertem Faktor V.

3. Zusammensetzung nach Anspruch 1 oder 2, dadurch gekennzeichnet, daß sie Protein C und/oder aktiviertes Protein C enthält.

4. Verwendung von Faktor V als natives Protein, dessen Derivat und/oder Fragment in Kombination mit Protein S und/oder einer Protein S haltigen pharmazeutischen Zusammensetzung zur Vorbeugung und Behandlung von Blutgerinnungsstörungen.

5. Verwendung nach Anspruch 4, dadurch gekennzeichnet, daß der Faktor V im wesentlichen frei von aktiviertem Faktor V verwendet wird.

6. Verwendung nach einem der Ansprüche 4 oder 5, dadurch gekennzeichnet, daß zusätzlich Protein C und/oder aktiviertes Protein C verwendet wird.

7. Verwendung nach einem der Ansprüche 4 bis 6, dadurch gekennzeichnet, daß die Komponenten gemeinsam, gleichzeitig oder hintereinander in beliebiger Reihenfolge verwendet werden.

8. Verwendung von Faktor V als natives Protein, dessen Derivat und/oder Fragment in Kombination mit Protein S zur Herstellung einer pharmazeutischen Zusammensetzung zur Vorbeugung und Behandlung von Blutgerinnungsstörungen.

9. Verwendung nach Anspruch 8, dadurch gekennzeichnet, daß der Faktor V im wesentlichen frei von aktiviertem Faktor V eingesetzt wird.

10. Verwendung nach Anspruch 8 oder 9, dadurch gekennzeichnet, daß zusätzlich Protein C und/oder aktiviertes Protein C zur Herstellung der pharmazeutischen Zusammensetzung eingesetzt wird.

11. Zusammensetzung nach einem der Ansprüche 1 bis 4 bzw. Verwendung nach einem der Ansprüche 5 bis 10, dadurch gekennzeichnet, daß der Faktor V als natives Protein, dessen Derivat und/oder Fragment in einer Art und Menge enthalten bzw. verwendet wird, die keine prokoagulatorische Aktivität aufweist.

12. Zusammensetzung nach einem der Ansprüche 1 bis 4 bzw. Verwendung nach einem der Ansprüche 5 bis 10, dadurch gekennzeichnet, daß die Gesamtmenge an als Faktor V wirkender Substanz in einem molaren Verhältnis von 1:2 bis 1:100, vorzugsweise 1:5 bis 1:50, zu Protein vorliegt.

13. Set zur Herstellung einer pharmazeutischen Zusammensetzung nach einem der Ansprüche 1 bis 4 oder zur Verwendung nach einem der Ansprüche 5 bis 10, umfassend einen Behälter (a), der den Faktor V, dessen Derivat und/oder Fragment enthält, und einen zweiten Behälter (b), der Protein S enthält, wobei die Behälter gegebenenfalls zur Herstellung der pharmazeutischen Zusammensetzung oder zur Verwendung erforderliche Hilfs-, Träger und/oder Zusatzstoffe enthalten können.
